# EUROPEAN PATENT SPECIFICATION

(11) **EP 4 452 372 B1**
(45) Date of publication and mention of the grant of the patent: **19.11.2025**
(21) Application number: 22840620.3
(22) Date of filing: 19.12.2022
(51) Int. Cl.: A61M 11/02, A61M 15/00

(54) **POWDER INHALER**
PULVERINHALATOR
INHALATEUR DE POUDRE

(30) Priority: 21.12.2021 EP 21216517
(43) Date of publication of application: 30.10.2024
(73) Proprietor: Chiesi Farmaceutici S.p.A., 43122 Parma (IT)
(72) Inventor: DI CASTRI, Marco, Parma (IT); MULAS, Giuseppe Antonio, Parma (IT); BOTTINI, Sara, Parma (IT); TWEEDIE, Alan, Parma (IT); HAYTON, Paul Graham, Parma (IT); VARVOUNIS, Odysseas Michail, Parma (IT); HIGGINS, Daniel David, Parma (IT)
(74) Representative: PGA S.p.A.
(86) International application number: PCT/EP2022/086690
(87) International publication number: WO 2023/117932

(56) References cited:
- EP-A1- 1 386 630
- WO-A1-2004/080606
- US-A1- 2009 260 626
- US-A1- 2012 017 902
- US-A1- 2016 095 990
- US-A1- 2021 316 091
- US-B2- 8 508 385

## Description

### Technical field of the invention

The present invention relates to a dry powder inhaler, i.e. a device for dispensing a powdered medicament preparation by inhalation. The device is in particular a portable, multiple-dose, breath activated dry powder inhaler without propellant gas, equipped with a metering device which dispenses doses from a medicament container.

### Background art

Inhalers are hand-held portable devices that deliver medication directly to the lungs. One class of inhalers is passive dry powder inhalers ("DPI"). A passive DPI is a patient driven device wherein the action of breathing in through the device draws the powder formulation into the respiratory tract. DPIs are well recognized as a method of drug delivery to the lung for treatment of pulmonary and systemic diseases. They can generally be divided in: i) single-dose (unit-dose) inhalers, for the administration of an individual dose of the active ingredient/s (in capsule or blister) loaded by the patient immediately before use; ii) pre-metered multi-dose inhalers containing a series of blisters or capsules with the active ingredient/s formulation or iii) reservoir inhalers containing a larger amount of the powder formulation of active ingredient/s, corresponding to multiple doses, which is metered from a storage unit just before inhalation.

Document WO 2004/012801, by the same Applicant, discloses a powder inhaler comprising a casing with a lower shell and an integral cover pivotably coupled to the lower shell. The lower shell delimits a mouthpiece and the integral cover is movable between a closed position in which the mouthpiece is enclosed and hidden by the integral cover and an open position in which the mouthpiece is exposed for use. The lower shell houses a container for storing a powdered medicament, a metering member having a dosing recess to be filled with a dose of the powdered medicament and an inhalation channel in communication with the mouthpiece. The powder inhaler further comprises a protective member which is slidingly moveable on the metering member between a closed position, in which the protective member covers the dosing recess of the metering member if the metering member is in an inhalation position, and an open position, in which the protective member exposes the dosing recess thereby enabling inhalation of the dose of the powdered medicament contained in the dosing recess. The protective member is coupled to an inhalation actuated mechanism in such a manner that the inhalation actuated mechanism moves the protective member from its closed position to its open position if there is an inhalation suction force exerted by a user which exceeds a predetermined level. Document WO 2016/000983, by the same Applicant, discloses a powder inhaler similar to the one of WO 2004/012801.

Document WO 92/09322 discloses a propellant-free inhalation device with a supply of pulverized medical substance. The device is provided with a mouthpiece for active inhalation and an air channel to distribute a dose discharged from a dosing chamber in the flow of breathing air. The mouthpiece can be closed with a cover which comprises detent noses to engage into air intakes and subsequently close the same and take care of a safe sealing of the device.

Document US 2012/0017902 discloses a nasal delivery device comprising a cap provided for covering a discharge aperture of a discharge member of the device during non-use. A seal protrusion extends from an inner surface of the cap to engage the discharge member.

Document US 8,508,385 discloses a metering device for inhaling a powder substance comprising a closure cap.

Document US 2016/0095990 discloses a deformable mouthpiece cover made from an elastomeric material for a dry powder inhaler.

Document US 2021/0316091 discloses a dry powder inhaler with a mouthpiece cover tightly closing an air inlet, when in closed position.

A formulation for powder inhalers is commonly a powder blend of one or more active ingredients dispersed in a pharmacologically inert bulk solid, comprising a physiologically acceptable diluent, such as lactose, and optional additional excipients such as lubricants. The particle size of inhalable active ingredients should be optimized to deliver the drug deep into the lung to achieve efficacy. This efficacious particle size typically lies between 1-6 µm whereas larger particles, in the range 6-10 µm, tend to be deposited in the upper airways without reaching the site of action in the lower airways.

It is well known that stability of the powder as well as the aerosol performances could be affected by environmental conditions, humidity in particular. Therefore, it is desirable to control the humidity within powder inhalers and in particular in reservoir DPIs. There is the need of more efficacious systems to protect from humidity, in particular but not solely, when the drug-containing powder formulation from reservoir DPIs are intended for being stored and used in sub-tropical and tropical countries.

### Summary

It is an object of the present invention to eliminate the above drawbacks of hitherto known powder inhalers and to provide a powder inhaler with an improved protection from environmental conditions, like humidity, temperature, light, pressure.

In particular, it is an object of the present invention to provide a powder inhaler which is able to control the humidity inside the inhaler itself.

It is object of the present invention to prevent or at least to limit the entry of atmospheric moisture inside the inhaler and in particular inside the container storing the powdered medicament during distribution and storage at a pharmacy (i.e. before delivering the inhaler to the user) and also after delivering the inhaler to the user, during the time period (e.g. days, months) when the user uses the inhaler.

At least one of the above objects is substantially achieved by a powder inhaler according to the present invention, as defined by the appended claims.

The word "more deformable" referred to the sealing element in this description and claims means that, when the sealing element engages the mouthpiece and/or the casing, said sealing element deforms more than the mouthpiece and/or the casing so as to adapt its shape to that of the mouthpiece and/or of the opening of the mouthpiece and/or the casing and copy its/their shape. In other words, the sealing element is more flexible and softer than the mouthpiece and/or the casing in order to mimic the external geometry of the mouthpiece and/or casing, to fill the gaps of the mouthpiece/casing and avoid that humidity could enter through these gaps.

The invention allows to improve the stability of the powder and the aerosol performances (i.e. the capability of delivering the drug deep into the lung to achieve efficacy) by protecting said powder from the environmental conditions, particularly from humidity.

In particular, the invention allows to prevent or at least limit the entry of atmospheric moisture inside the inhaler through the mouthpiece and/or the air channel/s when the cover of the inhaler is closed.

Therefore, the invention allows to protect the powder during the period when the inhaler is in use but is closed and stored in a bag, in a pocket, in the open air, in a drawer or in a furniture or other place between inhalations.

### Description of the drawings

Fig.1 shows a 3D view of a powder inhaler according to the present invention in an open configuration;
Fig.2 shows an enlarged view of a portion of the powder inhaler of figure 1;
Fig.3 shows a 3D view of one element of the powder inhaler of figure 1;
Fig.4 shows another 3D view of the element of figure 3;
Fig.5 is a plan view of one side of the element of figure 3;
Fig.6 is a plan view of another side of the element of figure 3;
Fig.7 is a side view of the element of figure 3;
Fig.8 is a section view of the element of figure 3 taken along lines VIII-VIII of figure 5;
Fig.9 is a section view of the powder inhaler of figure 1 provided with the element of figures 3 to 8;
Fig.10 is a chart showing a cumulative weight over time due to moisture absorption in a standard powder inhaler compared with the invention; and
Fig.11 is a chart showing a differential weight over time due to moisture absorption of two different embodiments of the invention.

### Detailed description

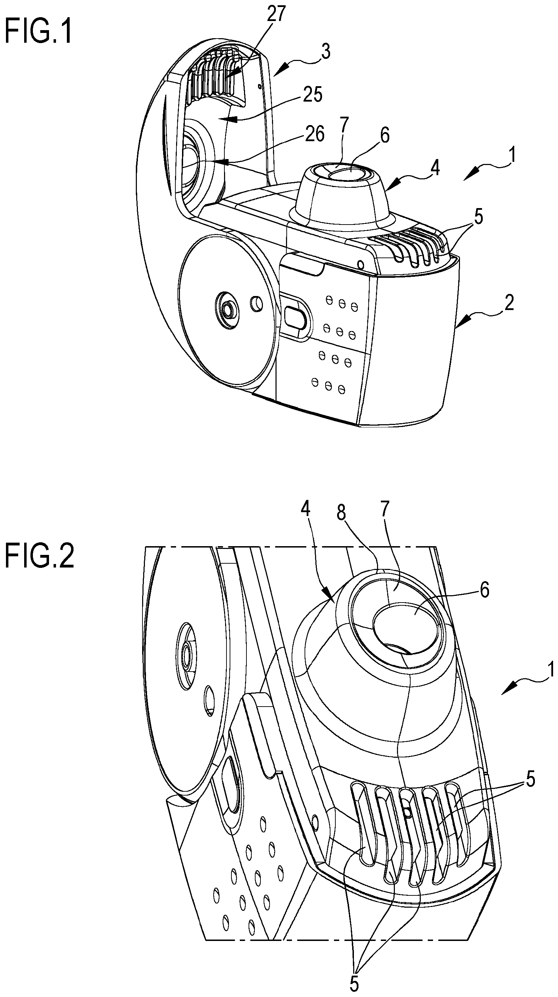

With reference to the appended drawings, Figures 1, 2 and 9 show a powder inhaler 1 according to the present invention. According to the example embodiment disclosed in the following description, the powder inhaler 1 may be substantially the same as or similar to the one disclosed in document WO 2004/012801 or in document WO 2016/000983 of the same Applicant.

The powder inhaler 1 comprises a casing 2 and a cover 3 being pivotably or rotatably coupled to the casing 2. As can be taken from Fig. 1, the cover 3 can be opened to reveal a mouthpiece 4 through which a user can inhale a powdered medicament. At an upper front side of the mouthpiece 4, air inlets 5 are formed in the casing 2.

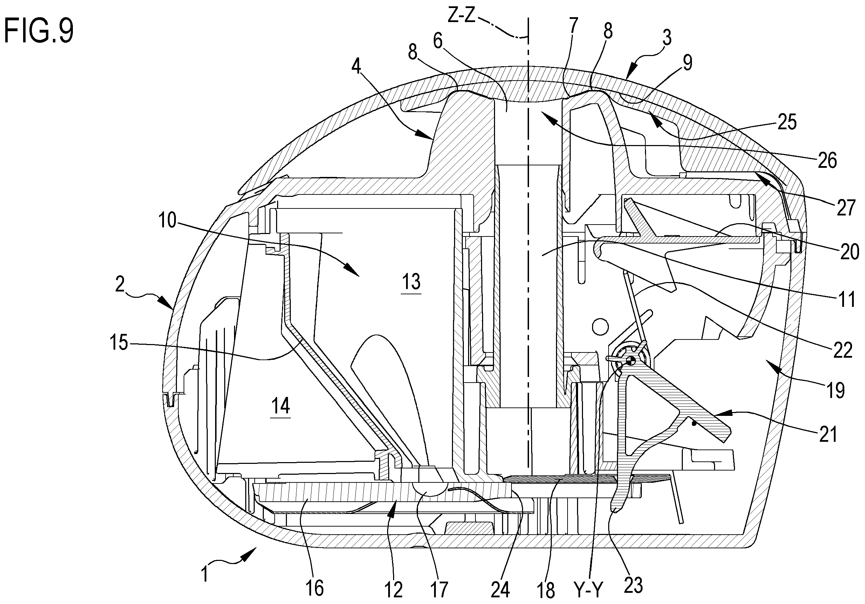

The casing 2 is a closed shell made of a rigid thermoplastic material (e.g. PP, PE, PVC, ABS, PC, PET, Nylon, etc.) and comprises lateral sides, an upper side and a lower side (upper and lower with respect to the orientation of the powder inhaler 1 of figures 1 and 9). The mouthpiece 4 protrudes from the upper side and has an external shape of truncated cone tapering towards an opening 6 fashioned in a top portion (smaller base) of the mouthpiece 4. The smaller base delimits a concave area 7 surrounded by a rim 8 (see Figures 1, 2 and 9) and the opening 6 is made in said concave area.

The concave area 7 and the rim 8 have an oval outline and the opening 6 has a circular outline. In other embodiment, not shown, the shape of the opening 6 and of the top portion of the mouthpiece 4 may be others (e.g. circular or elliptical). As can be seen in Figures 1 and 2, the opening 6 is off-center with respect to the oval outline of the concave area 7.

The air inlets 5 are slit shaped and parallel to each other. These slit shaped air inlets 5 are formed partly on the upper side and partly on a corner of the casing 2 connecting the upper side to one of the lateral sides (Figures 1 and 2).

The cover 3 is an open shell made of plastic material (e.g. the same material of the casing 2) and having an outer surface and a substantially concave inner surface 9. The inner surface 9 comprises two lateral sides and one bottom side. The cover 3 is hinged to the casing 2 and can be rotated between a closed position, shown in Figure 9, in which the cover 3 encloses the mouthpiece 4, and an open position, shown in Figure 1, in which the cover 3 is spaced from the mouthpiece 4 to expose said mouthpiece 4 for use.

The powder inhaler 1 comprises a container 10 for storing a powdered medicament, an inhalation channel 11 connected to the opening 6 of the mouthpiece 4 and a dispensing device 12. The inhalation channel 11 has a first opening connected to the mouthpiece 4 and a second opening, opposite with respect to the first opening. As shown in Figure 9, all these elements are housed inside the casing 2.

As is shown in Figure 9, the container 10 is a container with integral desiccant. The container 10 comprises a medicament chamber 13 storing the powdered medicament and a desiccant chamber 14 storing a desiccant for absorbing moisture that may have entered the medicament chamber 13. The desiccant chamber 14 is separated from the medicament chamber 13 by a separate permeable membrane 15. This permeable membrane 15 is of a different permeability than the permeability between either the desiccant or the medicament to the outside environment. The permeability of the membrane 15 can be achieved, for example, by making it of a different material and/or a thinner section than the main body of the container 10. Foils may be used to seal both the medicament chamber 13 and the desiccant chamber 14.

The container 10, in particular the medicament chamber 13, is filled or is configured to be filled with an amount of powder medicament corresponding to a plurality of doses, e.g. up to 100 - 200 doses. For instance, the powdered medicament is a pharmaceutical composition comprising a pharmaceutically acceptable salt of formoterol, e.g. in combination with a pharmaceutically acceptable salt of glycopyrronium and/or beclometasone dipropionate (BDP).

The desiccant is contained in a bag able of being inserted in the desiccant chamber 14 or the desiccant is in the form of a single tablet able of being inserted in the desiccant chamber 14. The desiccant is or comprises molecular sieves made of a material with pores of uniform size, for instance alkaline salts of aluminosilicates, called zeolites, or aluminophosphates or porous glass or active carbon or artificial zeolites. The molecular sieves are configured to absorb small molecules such as molecules of water.

The dispensing device 12 comprises a metering device 16 having a dosing recess 17. The metering device 16 shown in Figure 9 is a shuttle shaped like a plate and provided with said dosing recess 17. The metering device 16 is movable, with respect to the container 10 and with respect to the inhalation channel 11, between an idle state, in which a dosing recess 17 is in communication with an opening of the container 10 so as to be filled with a dose of the powdered medicament, and a triggered state, in which the dosing recess 17 is in communication with the inhalation channel 11 for enabling inhalation of the dose of the powdered medicament contained in the dosing recess 17 through the mouthpiece 4.

The shuttle is slidingly moveable between a filling position (Fig. 9) and an inhalation position (not shown). The filling position corresponds to the idle state of the metering device 16, in which the dosing recess 17 is in alignment with the opening of the container 10 so as to be filled with the dose of the powdered medicament. The inhalation position corresponds to an armed state which will be detailed later and to the triggered state of the metering device 16, in which the dosing recess 17 is in alignment with the inhalation channel 11.

The shuttle is mechanically coupled to the cover 3 such that an opening of the cover 3 beyond a range of rotational movement from the closed position causes the shuttle to move from the filling position to the inhalation position. Closing of the cover 3 causes the shuttle to move back from the inhalation position to the filling position. Fig.9 shows the cover 3 in the closed position and the shuttle in the filling position. The metering device 16 further comprises a protective member 18 provided between the shuttle and the inhalation channel 11. The protective member 18 is a plate arranged between the second opening of the inhalation channel 11 and the shuttle.

The protective member 18 is parallel with respect to the shuttle and is slidingly movable on the shuttle between a closed position and an open position. In the closed position, the protective member 18 is shifted backwards towards the second opening of the inhalation channel 11 and towards the container 10. In the closed position, a rear part of the protective member 18 may at least in part close the second opening of the inhalation channel 11. In the open position, the protective member 18 is shifted forward towards a wall of the casing 2. In the open position, a rear part of the protective member 18 leaves the second opening of the inhalation channel 11 open.

The protective member 18 is in the closed position when the shuttle is in the filling position. The protective member 18 may be moved between the closed position and the open position when the shuttle is in the inhalation position. Therefore, the metering device 16 is configured to take the three different states cited above (idle, armed, triggered) and these states are determined by the positions of the shuttle and of the protective member 18. In the idle state, the shuttle is in the filling position and the protective member 18 is in the closed position. The protective member 18 does not cover the dosing recess 17. The dosing recess 17 is communication with the opening of the container 10 to receive the medicament dose. In the armed state, the shuttle is in the inhalation position and the protective member 18 is in the closed position. The protective member 18 covers the dosing recess 17. The protective member 18 prevents the powdered medicament contained in the dosing recess 17 from entering the inhalation channel 11 and being lost in case of rotation or movement of the inhaler in oblique position before the inhalation manoeuvre or if the user blows into the mouthpiece. In the triggered state, the shuttle is in the inhalation position and the protective member 18 is in the open position. The protective member 18 does not cover the dosing recess 17, thereby exposing the dosing recess 17 to the inhalation channel 11 so as to enable a user to inhale the dose of the powdered medicament contained in the dosing recess 17.

The powder inhaler 1 shown in the attached Figure 9 further comprises a breath or inhalation actuated mechanism 19 coupled to the protective member 18. The inhalation actuated mechanism 19 comprises an inhalation actuated member 20 shaped like a flap, a coupling member 21 and a resilient element 22 arranged on the coupling member 21.

The flap 20 is coupled to the protective member 18 through the coupling member 21 such that, if there is an inhalation suction force exceeding a predetermined value, the flap 20 is moved from a first position to a second position, thereby causing the protective member 18 to move from the closed position to the open position. The flap 20 is placed inside the casing 2 and close to the air inlets 5.

In the first position (Fig. 9), the flap 20 separates the air inlets 5 from the inhalation channel 11 and seats in a main airflow path. The flap 20 provides a resistance if the user blows into the device giving positive feedback. In the second position, not shown, the flap 20 is rotated with respect to the first position to open the air inlets 5 and to allow air flowing through the air inlets 5 into the inhalation channel 11 and out of the mouthpiece 4. The resilient element 22 is arranged such that said resilient element 22 holds the flap 20 in its first position. When the shuttle is pushed forward by opening the cover 3, the resilient element 22 is compressed and charged and the reset force exerted on the flap 20 is released, so that the flap 20 can pivot or rotate from the first position into the second position that is pivoted downward relative to the first position if there is a sufficient high inhalation suction force in the inhalation channel 11.

The flap 20 is hinged to the casing 2 in order to rotate between the first position and the second position around a respective rotation axis which is substantially perpendicular to a main axis Z-Z of the inhalation channel 11. The coupling member 21 is also hinged to the casing 2 in order to rotate between a respective first position and second position around a respective rotation axis Y-Y which is substantially perpendicular to the main axis Z-Z of the inhalation channel 11.

The coupling member 21 comprises an arm, not shown in Figure 9, protruding towards the flap 20 and engaged with the flap 20 such that the clockwise rotation of the flap 20 from the first position to the second position causes a counterclockwise rotation of the coupling member 21 from its respective first position towards its respective second position.

The coupling member 21 comprises a prolongation 23 engaging with an opening formed in the protective member 18 in order to move the protective member 18 from the closed position to the open position when the coupling member 21 moves from its respective first position to its respective second position and vice-versa.

The prolongation 23 of the coupling member 21 is also moveably arranged in a longitudinal opening 24 which is formed in the shuttle along its longitudinal direction, such that said prolongation 23 can freely move in the longitudinal opening 24, while a movement of the shuttle from the inhalation position to the filling position causes the prolongation 23 of the coupling member 21 to abut against an edge of the longitudinal opening 24 thereby moving the coupling member 21 back into its initial first position.

The internal mechanisms and functioning of the powder inhaler 1 disclosed here above are substantially the same as disclosed in document WO2021/105440, by the same Applicant.

The powder inhaler 1 may also comprise a dose counting unit, not shown in the embodiment of the attached drawings, contained into the casing 2 and coupled both to the inhalation actuated mechanism 19 and to the closure of the cover 3 after an efficacious inhalation has occurred. The casing 2 may also comprise a window or an opening for displaying the number of doses taken or the number of doses left in the container 10, this number being counted by the dose counting unit. For instance, the dose counting unit is the same or similar to the one disclosed in document WO 2004/012801.

According to the present invention, the powder inhaler 1 further comprises a sealing element 25 housed in the cover 3 (as shown in Figures 1 and 9).

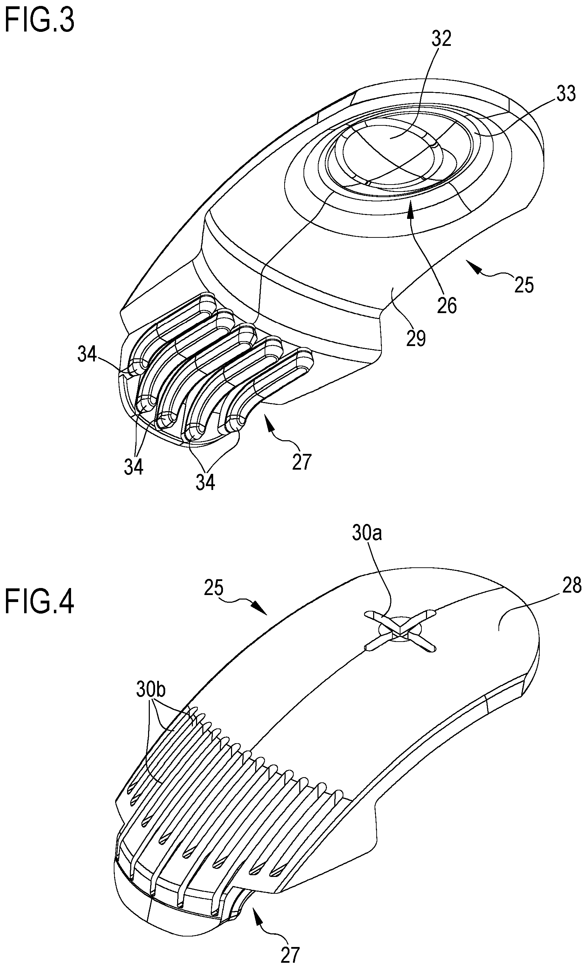

The sealing element 25 is coupled to the bottom side of the inner surface 9 of the cover 3 and may be over-molded to the cover 3 or press-fit in the cover and optionally glued to the cover 3 or mechanically connected to the cover 3 in another way. Different views of the standalone sealing element 25 are shown in the attached Figures 3 to 8.

The sealing element 25 is made of a material that is softer and more deformable than the material of the mouthpiece 4 and usually of the casing 2. The sealing element 25 is made of a soft or a medium soft material. In some embodiments, the sealing element 25 is made of silicone, e.g. PlatSil^{®} FS-20 mixed with PlatSil^{®} GEL-25. In other embodiments, the sealing element 25 may be of a thermoplastic elastomer (TPE), in particular TPE for medical - pharmaceutical applications, for instance styrene block copolymers (TPS (TPE-s)), thermoplastic polyolefin elastomers (TPO (TPE-o)), thermoplastic vulcanizates (TPV (TPE-v or TPV)), thermoplastic polyurethanes (TPU), thermoplastic copolyesters (TPC (TPE-E)), thermoplastic polyamides (TPA (TPE-A)), not classified thermoplastic elastomers (TPZ). Suitable TPE may be selected from those of medical - pharmaceutical grade belonging to any of the classes listed above.

For instance, the sealing element 25 has a hardness between 20 Shore A and 50 Shore A (e.g. 30 Shore A) and the casing 2 together with the mouthpiece 4 and the cover 3 have a hardness greater than 30 Shore D and lower than 100 Shore D (hard and extra-hard materials).

When the cover 3 is engaged with the casing 2 and closes the mouthpiece 4, a main portion 26 of the sealing element 25 is coupled to the opening 6 to tight close said opening 6 and an auxiliary portion 27 of the sealing element 25 is coupled to the air inlets 5 to tight close said air inlets 5. In the embodiment of Figure 9, when the cover 3 is in the closed position, portions of the second surface of the sealing element 25 other than the main portion 26 and the auxiliary portion 27 are spaced from the casing 2.

The main portion 26 and the auxiliary portion 27 mimic the external geometry of the top portion of the mouthpiece 4 and of the air inlets 5, in order to fill the gaps and avoid that humidity could enter through those gaps. Furthermore, the difference between the hardness of materials is such that, when the sealing element 25 engages the mouthpiece 4 and the casing 2 (closed position), said sealing element 25 deforms more than the mouthpiece 4 and the casing 2 so as to adapt its shape to that of the mouthpiece 4 and the casing 2 and copy their shape.

In the embodiment of the attached Figures, the sealing element 25 is made of a single body having a first substantially convex surface 28 coupled to the concave inner surface 9 of the cover 3 and a second surface 29 carrying the main portion 26 and the auxiliary portion 27. In other embodiments, not shown, the main portion 26 and the auxiliary portion 27 may be two separate parts.

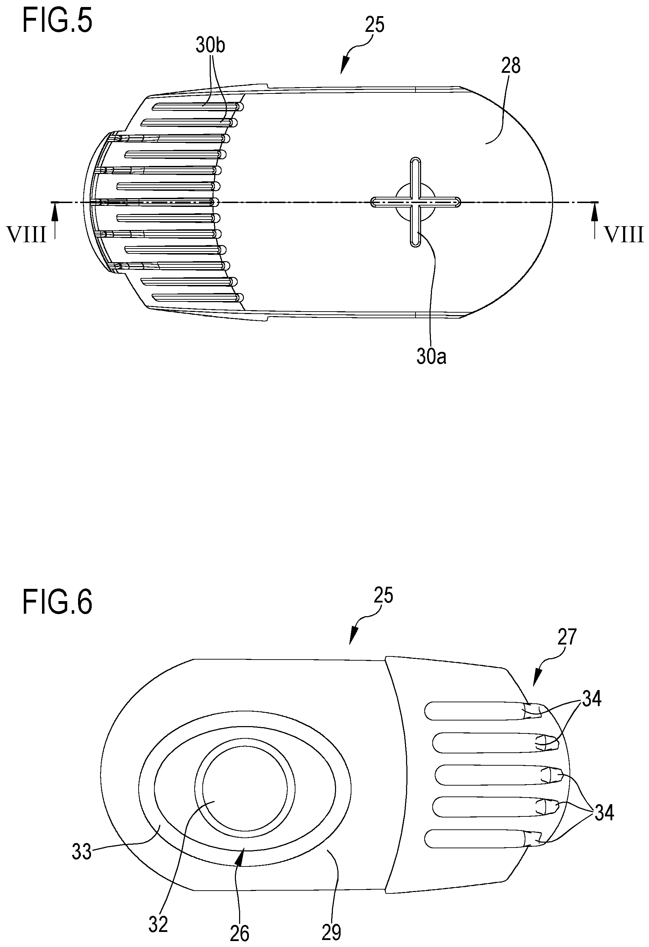

As represented in Figures 4 and 5 the first surface 28 of the sealing element 25 has indentations and/or recesses 30a, 30b configured to couple with elements/protrusions, not shown, of the cover 3 and/or to hold glue connecting the sealing element 25 to said cover 3. In the disclosed embodiment, the indentations and/or recesses 30a, 30b comprise a cross recess 30a accommodating a cross insert, not shown, part of the cover 3 and a plurality of parallel slits 30b accommodating parallel rail inserts, not shown, part of the cover 3.

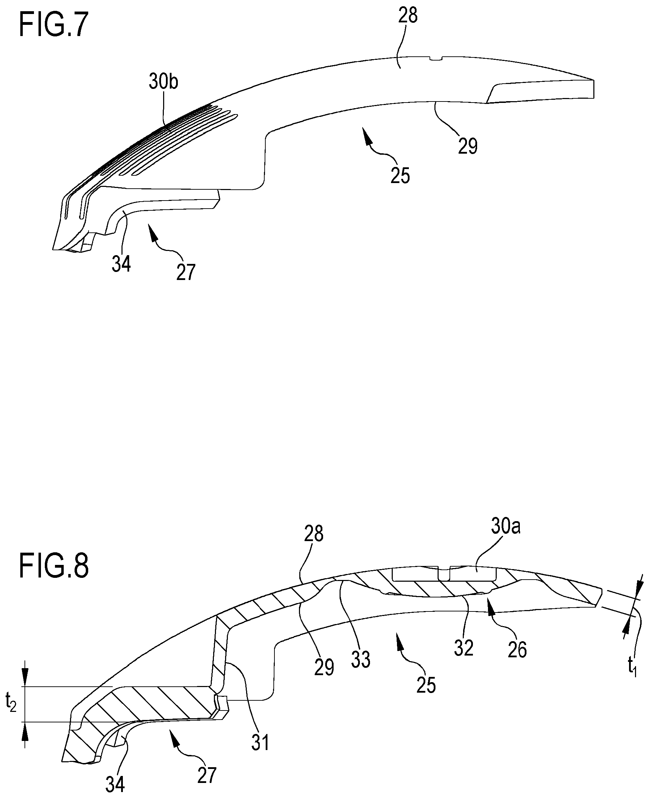

As shown in particular in Figure 8, a first part of the sealing element 25 in which the main portion 26 is fashioned is substantially flat and has a first average thickness "t1" of tenths of millimeter, e.g. 0.5 mm. A second part of the sealing element 25 forms a sort of step 31 with the first part and carries the auxiliary portion 27.

The main portion 26 of the sealing element 25 has a shape copying a shape of the concave area 7 and of the rim 8 of the mouthpiece 4 and to seal the opening 6 when the cover 3 is engaged with the casing 2 and closes the mouthpiece 4 (closed position of Figure 9). The main portion 26 comprises a convex dome shaped part 32 surrounded by an elliptical recess 33. The convex dome shaped part 32 is off-center with respect to the elliptical recess 33. In the closed position, the convex part 32 is centered on the opening 6 and copies in part a shape of the concave area 7 of the mouthpiece 3 while the rim 8 of the top portion of the mouthpiece 4 is accommodated in the elliptical recess 33.

The auxiliary portion 27 comprises a plurality of ribs 34 parallel to each other and each engageable in one of the slit shaped air inlets 5. The ribs 34 have a curved design to copy the shape of the slit shaped air inlets 5 in order to seal said slit shaped air inlets 5 when the cover 3 is in the closed position. As shown in Figure 8, the auxiliary portion 27 of the second part of the sealing element 25 has an average second thickness "t2" of about 2 mm.

A test was performed by comparing a powder inhaler as detailed above with no sealing element with two different powder inhalers provided with the sealing element 25 made of PlatSil^{®} FS-20 mixed with PlatSil^{®} GEL-25.

### Example

Two different embodiments of the invention were tested:
▪ with the sealing element 25 completely attached to the cover (over-molded);
▪ with the sealing element 25 fixed to the cover but not attached (press-fit).

Test conditions: 30°C and 75% RH.

The desiccant was removed from the desiccant chamber 14 and the medicament chamber 13 was filled with 3g of calcium chloride anhydrous to evaluate the moisture ingress in said medicament chamber 13.

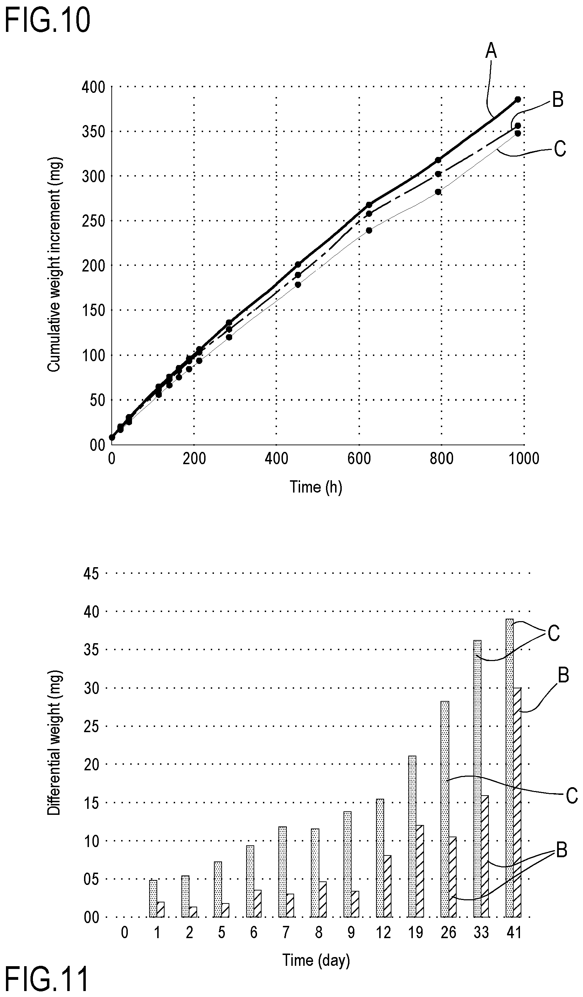

The chart of Figure 10 shows the increase over time (hours, abscissae) of the cumulative weight (mg, ordinates) due to moisture absorption of calcium chloride anhydrous in the medicament chamber 13, wherein:
A - no sealing element
B - over-molded sealing element
C - press-fit sealing element

After 41 days (984 h), both the embodiments B and C exhibit an improvement in the humidity protection.

The chart of Figure 11 shows the differential weight (mg, ordinates) increase over time (days, abscissae) of the two different embodiments of the invention (B - press-fit, C- over-molded)

The press-fit concept shows a saving of 39mg of water (10%) and the over-molded concept a saving of 30 mg (8%). Both the embodiments B and C show a cumulative effect (the saving of absorbed water increases over time).

The sealing element according to the technology may also be implemented in powder inhalers other than the one illustrated herein by way of example, e.g. with other shapes of the mouthpiece and/or of the cover and/or of the casing and/or with different couplings between the cover and the casing, different dispensing devices, etc.. The features of the sealing element of the technology (e.g. shapes, materials, etc.) will be designed/suitable for the respective powder inhaler in order to provide a correct sealing of the mouthpiece and of any air vent.

### List of parts

powder inhaler 1
casing 2
cover 3
mouthpiece 4
air inlets 5
opening 6
concave area 7
rim 8
inner surface 9
container 10
inhalation channel 11
dispensing device 12
medicament chamber 13
desiccant chamber 14
permeable membrane 15
metering device 16
dosing recess 17
protective member 18
breath or inhalation actuated mechanism 19
inhalation actuated member or flap 20
coupling member 21
resilient element 22
prolongation 23
longitudinal opening 24
a sealing element 25
main portion 26
auxiliary portion 27
first surface 28
second surface 29
indentations and/or recesses 30a, 30b
step 31
convex part 32
elliptical recess 33
ribs 34

## Claims

1. Powder inhaler, comprising:
a casing (2) having a mouthpiece (4) and housing an inhalation channel (11) connected to an opening (6) of the mouthpiece (4);
a container (10) for storing a powdered medicament and placed in the casing (2);
a dispensing device (12) placed in the casing (2) and configured to dispense unit doses of the powdered medicament from the container (10) to the inhalation channel (11) for inhalation through the mouthpiece (4);
a cover (3) engageable with the casing (2) to close the opening (6) of the mouthpiece (4);
wherein the cover (3) comprises a sealing element (25);
wherein the sealing element (25) comprises a material that is more deformable than a material of the mouthpiece (4);
wherein, when the cover (3) is engaged with the casing (2) and closes the mouthpiece (4), a main portion (26) of the sealing element (25) is coupled to the opening (6) to tight close said opening (6);
wherein the casing (2) has at least one air inlet (5) in fluid communication with the inhalation channel (11) to allow air intake at least when the user inhales through the mouthpiece (4);
**characterized in that**, when the cover (3) is engaged with the casing (2) and closes the mouthpiece (4), an auxiliary portion (27) of the sealing element (25) is coupled to the at least one air inlet (5) to tight close said at least one air inlet (5); wherein the material of the sealing element (25) is more deformable than a material of the casing (2).

2. The inhaler of claim 1, wherein the sealing element (25) comprises or is made of at least one of the following materials: silicone, thermoplastic elastomers (TPE).

3. The inhaler of any of claims 1 or 2, wherein the sealing element (25) has a hardness between 10 Shore A and 60 Shore A.

4. The inhaler of any of claims 1 to 3, wherein the sealing element (25) is over-molded to the cover (3) or press-fit in the cover (3) and glued to the cover (3) or mechanically connected to the cover.

5. The inhaler of any of claims 1 to 4, wherein the main portion (26) of the sealing element (25) has a shape copying a shape of the mouthpiece (4), wherein the main portion (26) of the sealing element (25) comprises a convex part (32) and wherein the convex part (32) is a dome shaped part.

6. The inhaler of claim 5, wherein a top portion of the mouthpiece (4) delimits a concave area (7) and the opening (6) is fashioned in said concave area (7); wherein the convex part (32) of the sealing element (25) has a shape copying at least in part a shape of the concave area (7).

7. The inhaler of claim 6, wherein the concave area (7) has a circular or oval or elliptical outline, wherein the opening (6) is off-center with respect to the circular or oval or elliptical outline of the concave area (7), wherein, when the cover (3) is engaged with the casing (2) and closes the mouthpiece (4), the convex part (32) of the sealing element (25) is centered on the opening (6).

8. The inhaler of claim 6 or 7, wherein the main portion (26) of the sealing element (25) comprises an annular or elliptical recess (33) surrounding the convex part (32) and accommodating, when the cover (3) is engaged with the casing (2) and closes the mouthpiece (4), a rim (8) of the top portion of the mouthpiece (4).

9. The inhaler of any of claims 1 to 8, wherein the auxiliary portion (27) of the sealing element (25) has a shape copying a shape of said at least one air inlet (5).

10. The inhaler of any of claims 1 to 9, wherein the at least one air inlet (5) is slit shaped and the auxiliary portion (27) of the sealing element (25) comprises at least one rib (34) engageable in the slit shaped air inlet (5).

11. The inhaler of claim 10, wherein the at least one slit shaped air inlet (5) is fashioned in part on a side of the casing (2) carrying the mouthpiece (4) and in part on one corner of the casing (2), wherein the at least one rib (34) has a curved design to follow the at least one slit shaped air inlet (5).

12. The inhaler of any of claims 1 to 11, wherein the sealing element (25) is made of a single body comprising the main portion (26) and the auxiliary portion (27).

13. The inhaler of any of the preceding claims 1 to 12, wherein the powder inhaler (25) comprises a desiccant chamber (14) placed inside the casing (2) and containing or configured to contain a desiccant, wherein the desiccant comprises molecular sieves.

14. The inhaler of claim 10, wherein the casing (2) has a plurality of slit shaped air inlets (5) and the auxiliary portion (27) comprises a plurality of ribs (34) each engageable in one of the slit shaped air inlet (5).

15. The inhaler of claim 13, wherein a permeable membrane (15) separates the desiccant chamber (14) from a medicament chamber (13) delimited by the container (10).

## Patentansprüche

1. Pulverinhalator, umfassend:
ein Gehäuse (2), welches ein Mundstück (4) aufweist und einen Inhalationskanal (11) aufnimmt, welcher mit einer Öffnung (6) des Mundstücks (4) verbunden ist;
einen Behälter (10) zum Lagern eines pulverförmigen Medikaments, welcher in dem Gehäuse (2) angeordnet ist;
eine Ausgabevorrichtung (12), welche in dem Gehäuse (2) angeordnet ist und welche dazu eingerichtet ist, Einheitsdosen des pulverförmigen Medikaments von dem Behälter (10) in den Inhalationskanal (11) zur Inhalation durch das Mundstück (4) auszugeben;
eine Abdeckung (3), welche mit dem Gehäuse (2) in Eingriff bringbar ist, um die Öffnung (6) des Mundstücks (4) zu schließen;
wobei die Abdeckung (3) ein Dichtungselement (25) umfasst;
wobei das Dichtungselement (25) ein Material umfasst, welches deformierbarer als ein Material des Mundstücks (4) ist;
wobei, wenn die Abdeckung (3) mit dem Gehäuse (2) in Eingriff ist und das Mundstück (4) schließt, ein Hauptabschnitt (26) des Dichtungselements (25) mit der Öffnung (6) gekoppelt ist, um die Öffnung (6) dicht zu schließen;
wobei das Gehäuse (2) wenigstens einen Lufteinlass (5) aufweist, welcher in Fluidkommunikation mit dem Inhalationskanal (11) ist, um eine Luftzufuhr zumindest dann zuzulassen, wenn der Nutzer durch das Mundstück (4) inhaliert;
**dadurch gekennzeichnet, dass**, wenn die Abdeckung (3) mit dem Gehäuse (2) in Eingriff ist und das Mundstück (4) schließt, ein Hilfsabschnitt (27) des Dichtungselements (25) mit dem wenigstens einen Lufteinlass (5) gekoppelt ist, um den wenigstens einen Lufteinlass (5) dicht zu schließen; wobei das Material des Dichtungselements (25) deformierbarer ist als ein Material des Gehäuses (2).

2. Inhalator nach Anspruch 1, wobei das Dichtungselement (25) wenigstens eines aus den folgenden Materialien umfasst oder aus diesen erzeugt ist: Silikon, thermoplastische Elastomere (TPE).

3. Inhalator nach Anspruch 1 oder 2, wobei das Dichtungselement (25) eine Härte zwischen 10 Shore A und 60 Shore A aufweist.

4. Inhalator nach einem der Ansprüche 1 bis 3, wobei das Dichtungselement (25) um die Abdeckung (3) umspritzt ist oder in die Abdeckung (3) eingepresst ist und an die Abdeckung (3) geklebt ist oder mechanisch mit der Abdeckung verbunden ist.

5. Inhalator nach einem der Ansprüche 1 bis 4, wobei der Hauptabschnitt (26) des Dichtungselements (25) eine Form aufweist, welche eine Form des Mundstücks (4) nachbildet, wobei der Hauptabschnitt (26) des Dichtungselements (25) einen konvexen Teil (32) umfasst, und wobei der konvexe Teil (32) ein kuppelförmiger Teil ist.

6. Inhalator nach Anspruch 5, wobei ein oberer Abschnitt des Mundstücks (4) einen konkaven Bereich (7) begrenzt und die Öffnung (6) in dem konkaven Bereich (7) geformt ist; wobei der konvexe Teil (32) des Dichtungselements (25) eine Form aufweist, welche eine Form des konkaven Bereichs (7) wenigstens teilweise nachbildet.

7. Inhalator nach Anspruch 6, wobei der konkave Bereich (7) ein kreisförmiges oder ovales oder elliptisches Profil aufweist, wobei die Öffnung (6) in Bezug auf das kreisförmige oder ovale oder elliptische Profil des konkaven Bereichs (7) exzentrisch ist, wobei, wenn die Abdeckung (3) in Eingriff mit dem Gehäuse (2) ist und das Mundstück (4) schließt, der konvexe Teil (32) des Dichtungselements (25) an der Öffnung (6) zentriert ist.

8. Inhalator nach Anspruch 6 oder 7, wobei der Hauptabschnitt (26) des Dichtungselements (25) eine ringförmige oder elliptische Vertiefung (33) umfasst, welche den konvexen Teil (32) umgibt und, wenn die Abdeckung (3) in Eingriff mit dem Gehäuse (2) ist und das Mundstück (4) schließt, einen Rand (8) des oberen Abschnitts des Mundstücks (4) unterbringt.

9. Inhalator nach einem der Ansprüche 1 bis 8, wobei der Hilfsabschnitt (27) des Dichtungselements (25) eine Form aufweist, welche eine Form des wenigstens einen Lufteinlasses (5) nachbildet.

10. Inhalator nach einem der Ansprüche 1 bis 9, wobei der wenigstens eine Lufteinlass (5) schlitzförmig ist und der Hilfsabschnitt (27) des Dichtungselements (25) wenigstens eine Rippe (34) umfasst, welche in den schlitzförmigen Lufteinlass (5) eingreifbar ist.

11. Inhalator nach Anspruch 10, wobei der wenigstens eine schlitzförmige Lufteinlass (5) teilweise an einer Seite des Gehäuses (2), welche das Mundstück (4) trägt, und teilweise an einer Ecke des Gehäuses (2) gebildet ist, wobei die wenigstens eine Rippe (34) eine gekrümmte Ausgestaltung aufweist, um dem wenigstens einen schlitzförmigen Lufteinlass (5) zu folgen.

12. Inhalator nach einem der Ansprüche 1 bis 11, wobei das Dichtungselement (25) aus einem einzelnen Körper erzeugt ist, welcher den Hauptabschnitt (26) und den Hilfsabschnitt (27) umfasst.

13. Inhalator nach einem der vorhergehenden Ansprüche 1 bis 12, wobei der Pulverinhalator (25) eine Trockenmittelkammer (14) umfasst, welche in dem Gehäuse (2) angeordnet ist und ein Trockenmittel enthält oder dazu eingerichtet ist, dieses zu enthalten, wobei das Trockenmittel Molekularsiebe umfasst.

14. Inhalator nach Anspruch 10, wobei das Gehäuse (2) eine Mehrzahl schlitzförmiger Lufteinlässe (5) aufweist und der Hilfsabschnitt (27) eine Mehrzahl von Rippen (34) umfasst, welche jeweils in einen der schlitzförmigen Lufteinlässe (5) eingreifbar sind.

15. Inhalator nach Anspruch 13, wobei eine permeable Membran (15) die Trockenmittelkammer (14) von einer Medikamentenkammer (13) trennt, welche durch den Behälter (10) begrenzt ist.

## Revendications

1. Inhalateur de poudre, comprenant :
un boîtier (2) ayant un embout buccal (4) et logeant un canal d'inhalation (11) raccordé à une ouverture (6) de l'embout buccal (4) ;
un récipient (10) de stockage d'un médicament en poudre et placé dans le boîtier (2) ;
un dispositif de distribution (12) placé dans le boîtier (2) et configuré pour distribuer des doses unitaires du médicament en poudre depuis le récipient (10) vers le canal d'inhalation (11) pour l'inhalation à travers l'embout buccal (4) ;
un couvercle (3) pouvant être engagé avec le boîtier (2) pour fermer l'ouverture (6) de l'embout buccal (4) ;
dans lequel le couvercle (3) comprend un élément d'étanchéité (25) ;
dans lequel l'élément d'étanchéité (25) comprend un matériau qui se déforme plus qu'un matériau de l'embout buccal (4) ;
dans lequel, lorsque le couvercle (3) est engagé avec le boîtier (2) et ferme l'embout buccal (4), une portion principale (26) de l'élément d'étanchéité (25) est accouplée à l'ouverture (6) pour fermer hermétiquement ladite ouverture (6) ;
dans lequel le boîtier (2) présente au moins un orifice d'entrée d'air (5) en communication fluidique avec le canal d'inhalation (11) pour permettre la prise d'air au moins lorsque l'utilisateur inhale à travers l'embout buccal (4) ;
**caractérisé en ce que**, lorsque le couvercle (3) est engagé avec le boîtier (2) et ferme l'embout buccal (4), une portion auxiliaire (27) de l'élément d'étanchéité (25) est accouplée à le au moins un orifice d'entrée d'air (5) pour fermer hermétiquement ledit au moins un orifice d'entrée d'air (5) ; dans lequel le matériau de l'élément d'étanchéité (25) peut plus se déformer qu'un matériau du boîtier (2).

2. Inhalateur selon la revendication 1, dans lequel l'élément d'étanchéité (25) comprend ou est constitué d'au moins l'un des matériaux suivants : silicone, élastomères thermoplastiques (TPE).

3. Inhalateur selon l'une quelconque des revendications 1 ou 2, dans lequel l'élément d'étanchéité (25) présente une dureté comprise entre 10 Shore A et 60 Shore A.

4. Inhalateur selon l'une quelconque des revendications 1 à 3, dans lequel l'élément d'étanchéité (25) est surmoulé au couvercle (3) ou ajusté par pression dans le couvercle (3) et collé au couvercle (3) ou mécaniquement raccordé au couvercle.

5. Inhalateur selon l'une quelconque des revendications 1 à 4, dans lequel la portion principale (26) de l'élément d'étanchéité (25) présente une forme copiant une forme de l'embout buccal (4), dans lequel la portion principale (26) de l'élément d'étanchéité (25) comprend une partie convexe (32) et dans lequel la partie convexe (32) est une partie en forme de dôme.

6. Inhalateur selon la revendication 5, dans lequel une portion supérieure de l'embout buccal (4) délimite une zone concave (7) et l'ouverture (6) est modelée dans ladite zone concave (7) ; dans lequel la partie convexe (32) de l'élément d'étanchéité (25) présente une forme copiant au moins en partie une forme de la zone concave (7).

7. Inhalateur selon la revendication 6, dans lequel la zone concave (7) présente un contour circulaire ou ovale ou elliptique, dans lequel l'ouverture (6) est décentrée par rapport au contour circulaire ou ovale ou elliptique de la zone concave (7), dans lequel, lorsque le couvercle (3) est engagé avec le boîtier (2) et ferme l'embout buccal (4), la partie convexe (32) de l'élément d'étanchéité (25) est centrée sur l'ouverture (6).

8. Inhalateur selon la revendication 6 ou 7, dans lequel la portion principale (26) de l'élément d'étanchéité (25) comprend un renfoncement annulaire ou elliptique (33) entourant la partie convexe (32) et logeant, lorsque le couvercle (3) est engagé avec le boîtier (2) et ferme l'embout buccal (4), un rebord (8) de la portion supérieure de l'embout buccal (4).

9. Inhalateur selon l'une quelconque des revendications 1 à 8, dans lequel la portion auxiliaire (27) de l'élément d'étanchéité (25) présente une forme copiant une forme dudit au moins un orifice d'entrée d'air (5).

10. Inhalateur selon l'une quelconque des revendications 1 à 9, dans lequel le au moins un orifice d'entrée d'air (5) est en forme de fente et la portion auxiliaire (27) de l'élément d'étanchéité (25) comprend au moins une nervure (34) pouvant être engagée dans l'orifice d'entrée d'air (5) en forme de fente.

11. Inhalateur selon la revendication 10, dans lequel le au moins un orifice d'entrée d'air (5) en forme de fente est façonné en partie sur un côté du boîtier (2) portant l'embout buccal (4) et en partie sur un coin du boîtier (2), dans lequel la au moins une nervure (34) présente une conception incurvée pour suivre le au moins un orifice d'entrée d'air (5) en forme de fente.

12. Inhalateur selon l'une quelconque des revendications 1 à 11, dans lequel l'élément d'étanchéité (25) est constitué d'un corps unique comprenant la portion principale (26) et la portion auxiliaire (27).

13. Inhalateur selon l'une quelconque des revendications précédentes 1 à 12, dans lequel l'inhalateur de poudre (25) comprend une chambre d'agent de dessiccation (14) placée à l'intérieur du boîtier (2) et contenant ou configurée pour contenir un agent de dessiccation, dans lequel l'agent de dessiccation comprend des tamis moléculaires.

14. Inhalateur selon la revendication 10, dans lequel le boîtier (2) présente une pluralité d'orifices d'entrée d'air (5) en forme de fente et la portion auxiliaire (27) comprend une pluralité de nervures (34) pouvant être chacune engagée dans l'un de l'orifice d'entrée d'air (5) en forme de fente.

15. Inhalateur selon la revendication 13, dans lequel une membrane perméable (15) sépare la chambre d'agent de dessiccation (14) d'une chambre de médicament (13) délimitée par le récipient (10).
